Europäisches Patentamt

European Patent Office

Office européen des brevets

(11) Numéro de publication: **0 247 958**
**A1**

## DEMANDE DE BREVET EUROPEEN

(21) Numéro de dépôt: **87430021.3**

(22) Date de dépôt: **22.05.87**

(51) Int. Cl.⁴: **A 61 B 5/20**
A 61 B 10/00

(30) Priorité: **30.05.86 FR 8607922**

(43) Date de publication de la demande:
**02.12.87 Bulletin 87/49**

(84) Etats contractants désignés: **DE ES FR GB IT**

(71) Demandeur: **Merlin, Jacques**
**24 Place Castellane**
**F-13006 Marseille (FR)**

(72) Inventeur: **Merlin, Jacques**
**24 Place Castellane**
**F-13006 Marseille (FR)**

(74) Mandataire: **Moretti, René et al**
**C/O Cabinet BEAU DE LOMENIE 14, rue Raphael**
**F-13008 Marseille (FR)**

(54) **Appareil pour mesurer la diurèse horaire.**

(57) Appareil pour mesurer la diurèse horaire comportant deux récipients dont l'un (2) est fixe et solidaire d'un support (1) et dont l'autre (3) relié audit récipient fixe est monté pivotant sur celui-ci, caractérisé en ce que le récipient (3) comporte deux tourillons creux coaxiaux (3c/3d) dont l'un (3d) pénètre à la partie supérieure (2b) du récipient fixe (2) et dont l'autre tourillon (3c) est relié à un tube (4) de connexion à une sonde (5) destinée à être placée sur un patient, de telle sorte que l'urine provenant du patient est recueillie dans le récipient pivotant (3) et en ce que l'axe (XX₁) des tourillons (3c/3d) est parallèle à la partie supérieure (3b) du récipient pivotant (3) et est incliné, par rapport à l'horizontale, du côté du récipient fixe (2) dans le but de favoriser l'écoulement de l'urine dans le récipient fixe (2) lors du basculement vers le haut du récipient pivotant (3) et également éviter toute remontée d'urine dans le tube de connexion (4).

FIG.1

EP 0 247 958 A1

**Description**

## APPAREIL POUR MESURER LA DIURESE HORAIRE

La présente invention a pour objet un appareil pour mesurer la diurèse horaire.

Le secteur technique de l'invention est celui des appareils médicaux, encore appelés urimètres ou autres appareils pour la mesure de tous autres fluides physiologiques.

Les urimètres connus se composent généralement d'un récipient amovible porté par un support comportant des moyens d'accrochage pour fixer l'appareil à un lit ou à tout autre meuble. La sonde est raccordée à un tube de connexion, lui-même fixé audit récipient.

La quantité d'urine émise par le patient est recueillie dans ledit récipient et est mesurée périodiquement. A chaque lecture du volume d'urine, le récipient est vidangé pour évacuer l'urine dans une poche à urine.

La présente invention vise à apporter des perfectionnements à de tels urimètres.

L'objectif à atteindre est un appareil pour mesurer la diurèse horaire permettant de simplifier et de réduire, de façon importante, les manipulations du personnel hospitalier en accélérant la vidange de l'urine et la prise de prélèvement pour échantillonner les urines.

Cet objectif est atteint par l'appareil pour mesurer la diurèse horaire comportant deux récipients dont l'un est fixe et solidaire d'un support et dont l'autre, relié audit récipient fixe, est monté pivotant sur celui-ci, caractérisé en ce que le récipient comporte deux tourillons creux coaxiaux dont l'un pénètre à la partie supérieure du récipient fixe et dont l'autre tourillon est relié à un tube de connexion à une sonde destinée à être placée sur un patient, de telle sorte que l'urine provenant du patient est recueillie dans le récipient pivotant et en ce que l'axe des tourillons est parallèle à la partie supérieure du récipient pivotant et est incliné, par rapport à l'horizontale, du côté du récipient fixe dans le but de favoriser l'écoulement de l'urine dans le récipient fixe lors du basculement vers le haut du récipient pivotant et également éviter toute remontée d'urine dans le tube de connexion. L'angle d'inclinaison desdits tourillons est compris entre 12° et 20°, de préférence 17°.

Les deux récipients sont d'une forme générale triangulaire et sont complémentaires, le récipient pivotant étant étroit à sa partie inférieure, le récipient fixe à sa partie supérieure et le tourillon, qui relie les deux récipients, est de plus grand diamètre et pénètre dans l'une des parois latérales du récipient fixe.

Ledit support comporte une butée parallèle à la partie supérieure du récipient pivotant pour limiter la course de celui-ci lors de son basculement.

La partie supérieure du récipient pivotant est demi-cylindrique et la butée entoure étroitement partiellement ladite partie de telle sorte que le récipient pivotant puisse être au plus basculé vers le haut selon un angle de 90°, pour permettre l'écoulement de l'urine dans le récipient fixe sans provoquer de contamination par remontée d'urine dans la sonde ou dans le tube de connexion reliant la sonde au récipient pivotant.

Le récipient fixe comporte, au droite de l'orifice, dans lequel pénètre ledit tourillon, un joint pour assurer l'étanchéité entre le tourillon et ledit orifice.

L'appareil comporte autour de tourillon, qui est relié au tube de connexion de la sonde, un palier en forme de cavalier, lequel est agrafé de façon amovible audit support pour réaliser l'articulation desdits récipients autour des deux tourillons.

Le récipient fixe comporte, à sa partie inférieure, au moins un orifice d'écoulement prolongé par une partie tubulaire destinée à être reliée à une poche à urine.

Dans une autre forme de réalisation, ledit récipient comporte, à sa partie inférieure, deux orifices prolongés par une partie tubulaire.

Ledit appareil comporte en outre un siphon à l'entrée du récipient pivotant pour créer une rétention de la dernière urine produite par le patient, lequel siphon comporte un site de prélèvement de cette urine. Ledit siphon est relié, par une de ses extrémités audit tube de connexion à la sonde et est engagé et fixé par son autre extrémité au tourillon creux de plus petit diamètre et pénètre à l'intérieur du récipient pivotant pour éviter toute contamination par remontée de microbes et également éviter toute remontée de liquide.

Ledit siphon comporte en outre, à sa partie la plus basse, un site de prélèvement constitué par un bossage percé pour recevoir l'embout d'une seringue, lequel bossage est orthogonal à ladite partie du siphon et s'étend entre les entrée et sortie tubulaires de celui-ci.

Dans le mode de réalisation où le récipient fixe comporte un second orifice, celui-ci est prolongé à l'intérieur du récipient par un tube de surverse, de telle sorte que le récipient assure la fonction d'une seconde chambre de mesure.

Le récipient pivotant, qui est relié par la sonde au patient, a pour fonction de recueillir l'urine émise par le patient et en mesurer le volume jusqu'à environ 250 cm3.

Le récipient fixe auquel est articulé le récipient pivotant sert à recueillir l'urine venant de celui-ci et à l'acheminer dans la poche à urine, ce qui permet, après lecture par l'infirmier, de l'urine émise en une heure par le malade, de la vidanger facilement et très rapidement par basculement du récipient pivotant, lequel est gradué pour permettre la mesure.

Le récipient fixe constitue une chambre intermédiaire entre le récipient pivotant et la poche à urine pour recevoir l'urine qui surverse dudit récipient pivotant, lequel récipient fixe peut également servir de réservoir supplémentaire pour mesurer des volumes plus importants, de l'ordre de 500 cm3.

Le récipient fixe est solidaire d'une plaque-support qui comporte, à sa partie supérieure, des moyens d'accrochage au lit du malade et à sa partie inférieure, des moyens pour y accrocher la poche à urine.

L'appareil comporte, en outre, un site de prélèvement pour échantillonner les dernières urines, lesquelles sont recueillies dans un siphon situé à l'entrée de l'urimètre, ce site étant adapté pour recevoir l'embout d'une seringue.

La forme triangulaire du récipient pivotant permet de mesurer les premiers centimètres cubes d'urine de façon très précise.

L'appareil selon l'invention est simple à l'emploi, très rapide à vidanger, hygiénique et précis. Il s'agit d'un système clos du fait qu'après mesure de la diurèse, l'appareil est basculé et l'urine évacuée vers la poche récolteuse, ceci sans interruption du système fermé. L'appareil est à nouveau et très rapidement prêt à l'emploi.

Du fait du montage du siphon solidaire du récipient pivotant, l'urine contenue dans ledit siphon est également évacuée lors du basculement du récipient pivotant.

D'autres avantages et les caractéristiques de l'invention ressortiront à la lecture de la description suivant d'un mode de réalisation d'un appareil pour mesurer la diurèse horaire en référence au dessin annexé sur lequel :

- la figure 1 est une vue en perspective d'un appareil selon l'invention, le récipient pivotant étant mis en position rabattue de mesure de l'urine émise par le malade;

- la figure 2 est une vue en perspective dudit appareil dont le récipient pivotant est représenté basculé en position de vidange;

- la figure 3 est une vue en coupe partielle de la partie supérieure dudit appareil;

- la figure 4 est une vue en coupe suivant la ligne IV IV de la figure 3;

- la figure 5 est une vue en élévation partielle de la partie supérieure du récipient pivotant;

- la figure 6 est une vue suivant la flèche F du siphon qui équipe le récipient pivotant;

- la figure 7 est une vue de dessus du siphon de la figure 6.

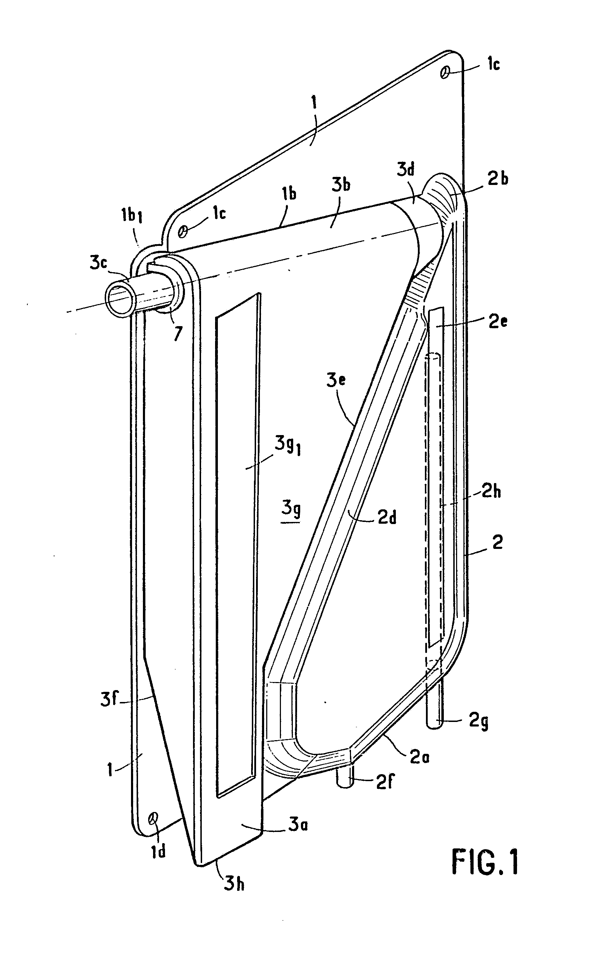

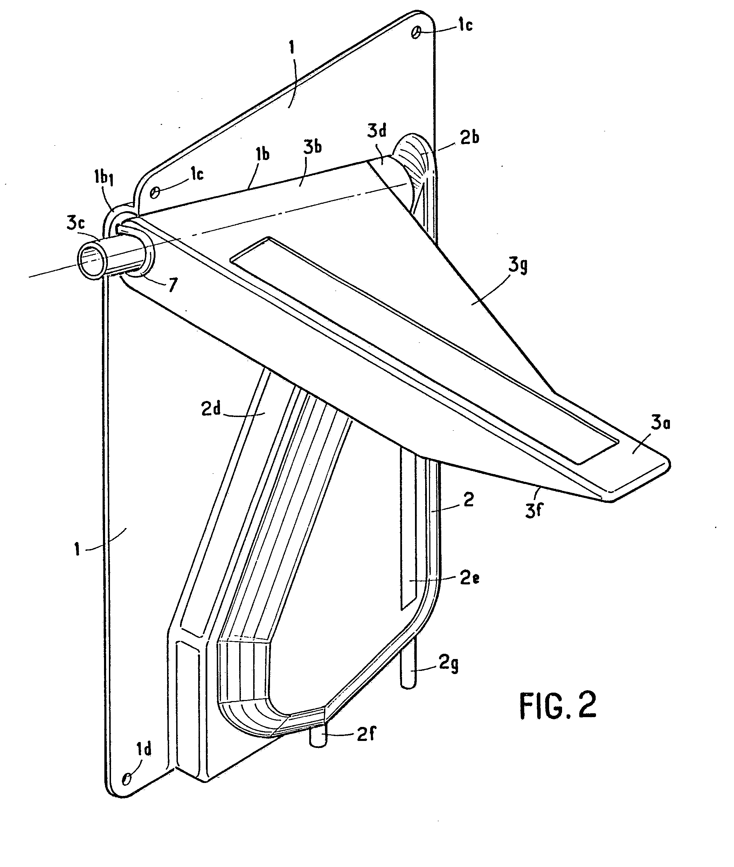

On se reporte d'abord aux figures 1 et 2 du dessin qui représentent le corps d'un appareil de mesure de la diurèse horaire selon l'invention. Celui-ci se présente sous la forme d'une unité comprenant une plaque-support 1 solidaire d'un récipient 2 d'une forme générale triangulaire, large à sa base 2a et étroit à sa partie supérieure 2b, lequel récipient est en relief sur la plaque-support 1.

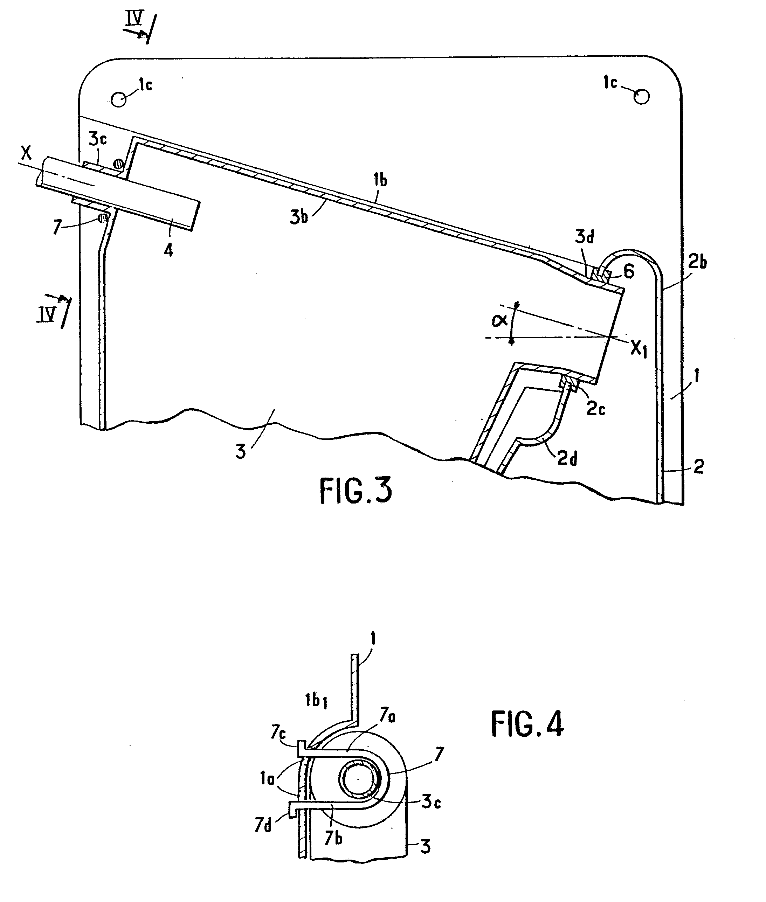

A ce récipient fixe 2, est articulé un autre récipient 3 d'une forme générale triangulaire, étroit à sa partie inférieure et large à sa partie supérieure 3b, de telle sorte que les deux récipients sont complémentaires et s'intègrent dans une figure géométrique adoptant la forme générale d'un trapèze. La partie supérieure 3b dudit récipient est demi-cylindrique et comporte, à l'une de ses extrémités, un tourillon creux 3c qui reçoit le tube de connexion 4 d'une sonde 5 (fig. 5) et à son autre extrémité, un autre tourillon creux 3d de plus grand diamètre que le tourillon 3c, qui pénètre dans un orifice 2c, réservé à la partie supérieure et dans la paroi latérale 2d dudit récipient fixe 2.

En position rabattue vers le bas du récipient pivotant 3, les deux récipients 2/3 sont juxtaposés de telle sorte que la paroi 2d du récipient 2 est sensiblement parallèle à la paroi latérale 3d du récipient 3, lesquelles parois sont à proximité l'une de l'autre.

Les tourillons 3c/3d sont coaxiaux et ont ainsi un axe commun $XX_1$ qui constitue l'axe d'articulation du récipient pivotant 3.

L'axe $XX_1$ est incliné du côté du récipient fixe 2 et forme avec l'horizontale un angle $\alpha$ compris entre 12° et 20°, de préférence 17°, de telle sorte que lorsque le récipient 3 est basculé vers le haut, l'urine contenue dans le récipient 3 soit rapidement évacuée dans le récipient 2 sans que l'opérateur n'ait à faire d'autre manoeuvre que de faire pivoter vers le haut le récipient 3.

Cette inclinaison permet également d'éviter toute remontée d'urine dans le tube de connexion 4.

Un joint en matière plastique souple 6, adoptant la forme d'un passage de paroi, assure l'étanchéité entre l'orifice 2c et le tourillon 3d.

Pour que le pivotement du récipient 3 s'effectue dans les conditions correctes, ledit récipient est guidé au droit du tourillon 3c par un palier en forme de cavalier 7, lequel est monté amovible sur la plaque-support 1 est est agrafé à cette dernière. Ledit cavilier comporte à l'extrémité libre de ses jambes 7a/7b des piètements 7c/7d s'étendant sur le côté des jambes 7a/7b et en opposition l'un de l'autre. Les piètements 7c/7d sont, de façon connue, passés dans des trous oblongs 1a réservés dans la plaque-support 1 et le palier 7 est agrafé du fait de l'élasticité du matériau qui le constitue, par exemple un acier à ressort ou tout autre matériau élastique.

Le débattement du récipient pivotant 3 est limité par une butée 1b située à la partie supérieure de l'appareil. Cette butée 1b est parallèle à la partie arrondie supérieure 3b du récipient 3 et contourne partiellement en $1b_1$ cette partie 3b selon un arc dont l'angle au centre est approximativement de 90°. Cette butée 1b a pour fonction de limiter le débattement du récipient 3 afin d'éviter toute remontée d'urine dans le tube de connexion 4 lors de la vidange.

Dans le but de faire la mesure des premiers centimètres cubes avec le maximum de précision, la partie étroite 3a du récipient 3 adopte la forme d'un biseau : la partie inférieure de la face arrière 3f et la face avant 3g du récipient 3 convergeant sur une ligne 3h qui délimite le récipient à sa partie extrême inférieure.

La face avant 3g comporte une zone en creux d'un contour rectangulaire $3g_1$ s'étendant sur la hauteur du récipient 3 dans laquelle zone sont réservées des graduations pratiquées dans la masse ou rapportées sur un support autocollant.

Le récipient 2 comporte également une zone graduée 2e qui s'étend sur la hauteur dudit récipient. Celui-ci comporte en outre, à sa partie inférieure, un orifice prolongé par une partie tubulaire 2f permettant de relier le récipient 2 à une poche à urine et éventuellement un deuxième orifice prolongé par une autre partie tubulaire 2g destinée à recevoir un tube 2h s'étendant à l'intérieur et sur la hauteur du réservoir 2 et constituant une surverse dans le cas où l'on utilise ledit récipient 2 comme chambre de mesure supplémentaire permettant de mesurer des volumes d'urine jusqu'à environ 500 cm3.

A cette fin, on obture temporairement le tube 2f et on crée une jonction au moyen d'un Y au-dessous de la partie obturée du tube 2f de façon à relier la surverse à la poche à urine.

La plaque-support est d'un contour rectangulaire à angles arrondis et comporte, à sa partie supérieure, deux trous 1c destinés à recevoir deux crochets (non représentés) pour suspendre l'appareil au lit du patient, et à sa partie inférieure deux trous 1d pour recevoir deux autres crochets pour relier l'appareil à une poche à urine quelconque, qui est ainsi suspendue auxdits crochets (not représentés).

Un seul trou 1d apparaît aux figures 1 et 2 du dessin.

Pour éviter toute contamination de la sonde 5 par remontée de microbes, le tube connecteur 4 est engagé dans le tourillon creux 3c du récipient 3 et pénètre par exemple entre dix et quinze millimètres à l'intérieur dudit récipient (fig. 3).

Une autre disposition est illustrée à la figure 5 du dessin. Selon ce mode de réalisation, le tube connecteur 4 est relié au récipient 3 par l'intermédiaire d'un siphon 8.

Ce siphon comporte deux prolongements tubulaires 8a/8b, situés dans un même plan et à la partie supérieure du siphon. Le tube connecteur 4 est engagé forcé dans l'entrée 8a, alors que le prolongement 8b, encore appelé "sortie" est engagé juste et fixé par exemple par collage dans le tourillon creux 3c du récipient 3.

Tel que cela est représenté à la figure 5, le prolongement 8b pénètre à l'intérieur du récipient 3 pour éviter toute contamination par remontée de microbes et pour éviter egalement toute remontée de liquide.

La partie la plus basse 8c du siphon 8 constitue une zone de rétention de la dernière urine émise par le patient et comporte un site de prélèvement constitué par un bossage percé longitudinalement 8d pour recevoir l'embout d'une seringue.

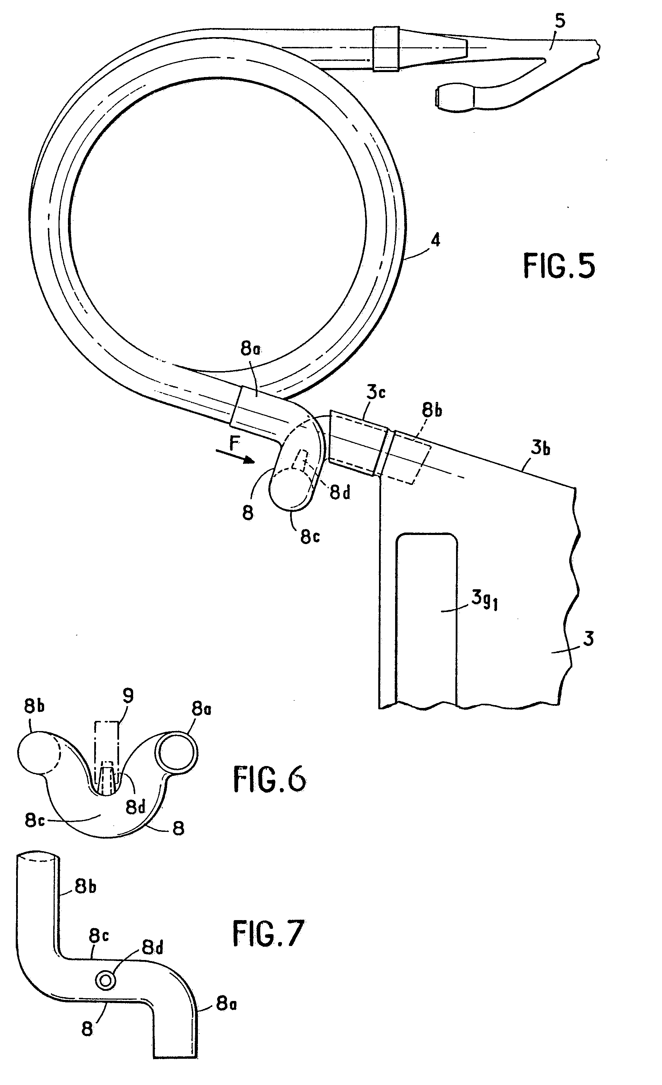

Ledit bossage 8d est orthogonal à la partie la plus basse 8c dudit siphon et s'étend entre lesdits prolongements tubulaires d'entrée et de sortie 8a/8b, son extrémité libre se situant approximativement au niveau de la partie inférieure desdits prolongements 8a/8b tel que cela peut être vu à la figure 6, dans le but d'éviter tout écoulement au moment où l'on pratique le prélèvement d'un échantillon d'urine.

Le bossage 8d reçoit un bouchon amovible 9.

Du fait de la conception de l'appareil, l'urine contenue dans le siphon est évacuée lors du basculement de récipient pivotant 3.

L'appareil selon l'invention est de préférence réalisé en matière plastique transparente ou translucide par exemple en chlorure de polyvinyle.

On notera toutefois que l'appareil qui vient d'être décrit comme étant conçu pour mesurer la diurèse horaire offre également la possibilité d'être utilisé pour la mesure de tout autre fluide physiologique autre que l'urine.

## Revendications

1. Appareil pour mesurer la diurèse horaire comportant deux récipients dont l'un (2) est fixe et solidaire d'un support (1) et dont l'autre (3) relié audit récipient fixe est monté pivotant sur celui-ci, caractérisé en ce que le récipient (3) comporte deux tourillons creux coaxiaux (3c/3d) dont l'un (3d) pénètre à la partie supérieure (2b) du récipient fixe (2) et dont l'autre tourillon (3c) est relié à un tube (4) de connexion à une sonde (5) destinée à être placée sur un patient, de telle sorte que l'urine provenant du patient est recueillie dans le récipient pivotant (3) et en ce que l'axe ($XX_1$) des tourillons (3c/3d) est parallèle à la partie supérieure (3b) du récipient pivotant (3) et est incliné, par rapport à l'horizontale, du côté du récipient fixe (2) dans le but de favoriser l'écoulement de l'urine dans le récipient fixe (2) lors du basculement vers le haut de récipient pivotant (3) et également éviter toute remontée d'urine dans le tube de connexion (4).

2. Appareil selon la revendication 1, caractérisé en ce que les deux récipients (2/3) sont d'une forme générale triangulaire et sont complémentaires, le récipient pivotant (3) étant étroit à sa partie inférieure (3a), le récipient fixe (2) à sa partie supérieure (2b) et en ce que le tourillon (3d), qui relie lesdits récipients (2/3) est de plus grand diamètre et pénètre dans l'une des parois latérales (2d) du récipient fixe (2).

3. Appareil selon l'une quelconque des revendications 1 et 2, caractérisé en ce que ledit support (1) comporte une butée (1b) parallèle à la partie supérieure (3b) du récipient pivotant (3) pour limiter la course de celui-ci lors de son basculement.

4. Appareil selon la revendication 3, caractérisé en ce que la partie supérieure (3b) du récipient pivotant (3) est demi-cylindrique et que la butée (1b) entoure étroitement partiellement en ($1b_1$) ladite partie (3b) de telle sorte que le récipient pivotant (3) puisse être au plus, basculé vers le haut selon un angle de 90° pour permettre l'écoulement de l'urine dans le récipient fixe (2) sans provoquer de contamination par remontée d'urine dans la sonde (5) ou dans le tube de connexion (4) reliant la sonde (5) au récipient pivotant (3).

5. Appareil selon la revendication 1, caractérisé en ce que l'angle $\alpha$ d'inclinaison des tourillons (3c/3d) est compris entre 12° et 20°, de préférence 17°.

6. Appareil selon l'une quelconque des revendications 1 à 5, caractérisé en ce que le récipient fixe (2) comporte au droit de l'orifice (2c), dans lequel pénètre ledit tourillon (3d), un joint (6) pour assurer l'étanchéité entre le tourillon (3d) et ledit orifice (2c).

7. Appareil selon l'une quelconque des revendications 1 à 6, caractérisé en ce qu'il comporte autour du tourillon (3c), qui est relié au tube (4) de connexion de la sonde (5), un palier (7) en forme de cavalier, lequel est agrafé de façon amovible audit support (1) pour réaliser l'articulation desdits récipients (2/3) autour des deux tourillons (3c/3d).

8. Appareil selon l'une quelconque des revendications 1 à 7, caractérisé en ce que le récipient fixe (2) comporte, à sa partie inférieure (2a), au moins un orifice d'écoulement prolongé par une partie tubulaire (2f) destinée à être reliée à une poche à urine.

9. Appareil selon la revendication 9, caractérisé en ce que ledit récipient (2) comporte, à sa partie inférieure (2a), deux orifices prolongés par une partie tubulaire (2f/2g).

10. Appareil selon l'une quelconque des revendications 1 à 9, caractérisé en ce qu'il comporte un siphon (8) à l'entree du récipient pivotant (3) pour créer une rétention de la dernière urine produite par le patient, lequel siphon (8) comporte un site de prélèvement de cette urine.

11. Appareil selon la revendication 10, caractérisé en ce que ledit siphon (8) est relié par une de ses extrémités audit tube (4) de connexion à la sonde (5) et est engagé et fixé par son autre extrémité au tourillon creux de plus petit diamètre (3c) et pénètre à l'intérieur du récipient pivotant (3) pour éviter toute contamination par remontée de microbes.

12. Appareil selon l'une quelconque des revendications 10 et 11, caractérisé en ce que ledit siphon (8) comporte, à sa partie la plus basse (8c), un site de prélèvement constitué par un bossage percé (8d) pour recevoir l'embout d'une seringue, lequel bossage (8d) est orthogonal à ladite partie (8c) du siphon et s'étend entre les entrée et sortie tubulaires (8a/8b) du siphon.

13. Appareil selon la revendication 9, caractérisé en ce que ledit second orifice (2g) est prolongé à l'intérieur du récipient (2) par un tube de surverse (2h), de telle sorte que le récipient (2) assure la fonction d'une seconde chambre de mesure.

0247958

FIG.1

0247958

FIG. 2

0247958

FIG.3

FIG.4

0247958

FIG.5

FIG.6

FIG.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 776 231  (HOLBROOK et al.) <br> * Colonne 1, lignes 8-23; colonne 2, lignes 57-64; colonne 4, ligne 34 - colonne 5, ligne 12; colonne 5, lignes 43-47; revendication 1; figures 1,7-10 * | 1 | A 61 B   5/20 <br> A 61 B  10/00 |
| A | | 2-4,6, 8 | |
| | --- | | |
| A | GB-A-1 588 102  (MEDIPLAST AB) <br> * Page 1, ligne 85 - page 2, ligne 30; figures 1,2 * | 1,2 | |
| | --- | | |
| A | US-A-3 919 455  (J.E. SIGDELL et al.) <br> * Abrégé; colonne 1, lignes 7-12; colonne 2, lignes 8-26; figure 1 * | 10,11 | **DOMAINES TECHNIQUES RECHERCHES (Int. Cl.4)** <br><br> A 61 B |
| | --- | | |
| A | US-A-3 906 935  (RAIA et al.) <br><br> * Colonne 2, ligne 28 - colonne 3, ligne 56; figures 1,2,2A * | 1,10-12 | |
| | ----- | | |

Le présent rapport de recherche a été établi pour toutes les revendications

| Lieu de la recherche | Date d'achèvement de la recherche | Examinateur |
|---|---|---|
| LA HAYE | 04-09-1987 | HUNT,B.W. |